# EUROPEAN PATENT SPECIFICATION

(11) **EP 0 480 189 B1**
(45) Date of publication and mention of the grant of the patent: **20.03.1996**
(21) Application number: 91115360.9
(22) Date of filing: 11.09.1991
(51) Int. Cl.: A61K 31/73

(54) **Pharmaceutical compositions for topical use comprising hyaluronic acid sodium salt and disinfectant substances**
Hyaluronsäurenatriumsalz und Desinfektionsmittel enthaltende pharmazeutische Zusammensetzungen zur äusserlichen Anwendung
Composition pharmaceutique à usage topique contenant du sel de sodium de l'acide hyaluronique et des désinfectants

(30) Priority: 05.10.1990 IT 2166290
(43) Date of publication of application: 15.04.1992
(73) Proprietor: ALTERGON S.A., CH-6900 Lugano (CH)
(72) Inventor: Donati Pedemonti, Elisabetta, I-22100 Como (IT); Lualdi, Paolo, I-22070 Grandate (IT)
(74) Representative: Gervasi, Gemma, Dr.

(56) References cited:
- GB-A- 1 240 545
- US-A- 4 736 024
- RIV. ITAL. PLAST. (ITALY), vol. 19, no. 4, 1987, PADOVA, pages 635-644; SILIPRANDI et al.: "Hyaluronic acid effects on deep burns healing"

## Description

### Prior art

Hyaluronic acid is an acid mucopolysaccharide which constitutes more than 50% of the basic substance of the dermis. Administration of exogenous hyaluronic acid determines an antiphlogistic and stimulating action on the granulation tissue, which accelerates cicatrization and re-epithelialization of lesions.

Pharmaceutical compositions comprising hyaluronic acid are known. For example, USA patent 4,736,024 describes compositions for topical application comprising hyaluronic acid and pharmacologically active substances, consisting for example of antibiotics such as erythromycin, gentamicin, neomycin etc.

European patent 0138572 describes pharmaceutical compositions comprising hyaluronic acid sodium or potassium salt for treating wounds and for use in the ophthalmic field.

The patent WO 84/04453 describes compositions for oral and dental treatment.

However, the problem of preparing compositions comprising hyaluronic acid and disinfectant substances has as yet not been solved. In this respect, various difficulties are encountered such as the poor compatibility between many disinfectant substances and hyaluronic acid, poor biocompatibility with the skin in prolonged topical use, etc.

For example, quaternary ammonium salts, guanidine and biguanidine derivatives and acridine derivatives precipitate the hyaluronic acid by complexing; mercury and phenol derivatives develop high toxicity and cause damage determined by their long retention time on the dermis; benzoic acid derivatives in association with antimycotics give rise to frequent allergic manifestations; iodine-based derivatives excessively colour the preparations and give rise to formulations of poor physico-chemical stability.

### Summary of the invention

We have now unexpectedly found that compositions comprising hyaluronic acid and disinfectant substances can be prepared having compatibility between the components and biocompatibility with the cutis, if the disinfectants are chosen from the group consisting of cresol derivatives, hexetidine and sulfadiazine silver salt.

Said compositions can be used with excellent results in the topical treatment of infected sores.

### Detailed description of the invention

The characteristics and advantages of the pharmaceutical compositions comprising hyaluronic acid sodium salt and disinfectant substances according to the present invention will be apparent in the course of the following detailed description.

In addition to the hyaluronic acid sodium salt and disinfectant substances the compositions also comprise pharmacologically acceptable excipients and diluents suitable for obtaining the desired pharmaceutical forms.

The disinfectant substances are chosen from the group consisting of cresol derivatives, hexetidine and sulfadiazine silver salt. The preferred cresol derivatives are chloroxylenol and dichloroxylenol.

The compositions can be prepared in various pharmaceutical forms suitable for use in topical treatment, such as oil-in-water emulsions, water-in-oil emulsions, hydrogels, pastes, ointments, lotions and powders for sprinkling.

The excipients used include for example hydroxypropylmethylcellulose, sorbitol, glycerin, polyoxyethylenated glycolyzed glycerides, polyethyleneglycol stearate, stearic acid, oleic acid decyl ester, caprylic and caproic acid esters, ethoxylated glycerides of palmitic and lauric acids, polymerized polyvinyl alcohol, self-emulsifying wax and non-denatured collagen.

The hyaluronic acid sodium salt content of the composition is between 0.1 and 0.5% by weight and the disinfectant substance content is between 0.1 and 2% by weight.

The composition pH depends on the pH of maximum activity of the disinfectant substance used but in all cases is between 4 and 7. The compositions in water-in-oil emulsion form have an HLB of 1-5 and the compositions in oil-in-water emulsion form have an HLB of 6-21.

Of the disinfectant substances suitable for use in preparing the compositions of the present invention, the preferred substance is sulfadiazine silver.

This substance is in fact the choice antimicrobic for disinfecting sores, burns, ulcerations etc. It is insoluble in water, it dissociates gradually in the organic liquids of the damaged surfaces and subsequently binds to the cellular walls of the infecting micro-organisms or of the epithelial cells.

This results in a slow and continuous antiseptic effect and prevents absorption of two ions (metal and organic).

In this respect, sulfadiazine silver does not produce argyric phenomena, and in addition the sulphamide part is poorly absorbed cutaneously, with the result that urinary elimination in 24 hours is of the order of 50 mg on average, this being a value considered far below the threshold for inducing renal damage.

The activity of sulfadiazine silver is not antagonized by p-aminobenzoic acid and in particular does not inhibit carbonic anhydrase, so preventing systemic acidosis.

Finally, it has a wide range of action, in particular against gram-negative micro-organisms, including Pseudomonas aeruginosa, Enterobacter cloacae and klebsiella, and against Staphylococcus aureus.

The association of hyaluronic acid and sulfadiazine silver is chemically and biologically compatible, and develops a synergic effect which results in rapid re-epithelialization of the damaged tissue in an aseptic environment.

The antibacterial activity of the association of hyaluronic acid sodium salt with sulfadiazine silver in a 1:5 ratio (as in Examples 4 and 5) was evaluated in vitro on certain bacterial strains and fungi, in comparison with the two components taken separately.

Although hyaluronic acid proved, as predictable, totally free of antibacterial effect, its association with the sulfadiazine silver led to an unexpected potentiation of the sulfadiazine activity.

This potentiation is of considerable interest from the applicative clinical-therapeutic aspect.

Table 1 below shows the MIC (minimum inhibiting concentration) values of sulfadiazine silver both alone (A) and in its association with hyaluronic acid sodium salt in the described ratio (B). The values are expressed as µg/ml of sulfadiazine silver.

**TABLE 1**

| STRAIN | MIC (µg/ml) | |
|---|---|---|
| | A | B |
| GRAM+ Staphylococcus Aureus ATCC 6538 | 62.5 | 15.26 |
| GRAM- Pseudomonas Aeruginosa ATCC 9027 | 62.5 | 31.25 |
| FUNGUS Candida Albicans ATCC 10231 | 31.25 | 15.62 |

Some examples are given hereinafter of topical pharmaceutical forms of the compositions according to the present invention. These examples are provided by way of illustration.

### EXAMPLE 1:

Composition in the form of an oil-in-water emulsion:

| | |
|---|---|
| Hyaluronic acid sodium salt | 0.1 - 0.5% by weight |
| Hexetidine | 0.05 - 0.5% by weight |
| Glycolyzed polyoxyethylenated glycerides | 1 - 5 % by weight |
| Polyethyleneglycol stearate and stearic acid | 2 - 4 % by weight |
| Sorbitol | 1 - 2 % by weight |
| Distilled water to make up to | 100 % by weight |

The emulsion has an HLB of 11 and a pH of 6.5-7. The disinfectant is dissolved in the lipid phase.

### EXAMPLE 2:

Composition in the form of a translucent hydrogel:

| | |
|---|---|
| Hyaluronic acid sodium salt | 0.1 - 0.5% by weight |
| Hexetidine | 0.1 - 0.3% by weight |
| Polymerized polyvinyl alcohol | 1 - 5 % by weight |
| NaOH solution to make up to | pH 7 |
| Sorbitol | 2 - 4 % by weight |
| Distilled water to make up to | 100 % by weight |

The disinfectant is present in microdispersed form.

### EXAMPLE 3:

Composition in the form of a water-in-oil emulsion:

| | |
|---|---|
| Hyaluronic acid sodium salt | 0.1 - 0.5% by weight |
| Chlorocresol | 0.1 - 1.0% by weight |
| Oleic acid decyl ester | 15 - 20 % by weight |
| Caprylic and caproic acid esters | 5 - 10 % by weight |
| Ethoxylated glycerides of palmitic and lauric acids | 20 - 30 % by weight |
| Distilled water to make up to | 100 % by weight |

The emulsion has an HLB of 4. The disinfectant is dissolved in the lipid phase.

### EXAMPLE 4:

Composition in the form of an oil-in-water emulsion:

| | |
|---|---|
| Hyaluronic acid sodium salt | 0.1 - 0.5% by weight |
| Sulfadiazine silver | 1 - 2 % by weight |
| Glycerin | 1 - 2 % by weight |
| Sorbitol | 1 - 3 % by weight |
| Polyethyleneglycol stearate | 8 - 12 % by weight |
| Oleic acid decyl ester | 4 - 6 % by weight |
| Self-emulsifying cream | 1.5 - 3 % by weight |
| Distilled water to make up to | 100 % by weight |

The emulsion has an HLB of 12 and a pH of 6-7. The disinfectant is micro-dispersed in the aqueous phase.

### EXAMPLE 5:

Composition in the form of a translucent hydrogel:

| | |
|---|---|
| Hyaluronic acid sodium salt | 0.1 - 0.5% by weight |
| Sulfadiazine silver | 1 - 2 % by weight |
| Glycerin | 2 - 7 % by weight |
| Non-denatured collagen | 1 - 4 % by weight |
| Sorbitol | 1 - 2 % by weight |
| Distilled water to make up to | 100 % by weight |

The hydrogel has a pH of 6-7. The disinfectant is present in micro-dispersed form.

## Claims

1. Pharmaceutical compositions for topical use comprising hyaluronic acid sodium salt and disinfectant substances chosen from the group consisting of cresol derivatives, hexetidine and sulfadiazine silver salt.

2. Compositions as claimed in claim 1, characterised in that said cresol derivatives are chloroxylenol and dichloroxylenol.

3. Compositions as claimed in claim 1, characterised by comprising as excipients and diluents hydroxypropylmethyl-cellulose, sorbitol, glycerin, polyoxyethylenated glycolyzed glycerides, polyethyleneglycol stearate, stearic acid, oleic acid decyl ester, caprylic and caproic acid esters, ethoxylated glycerides of palmitic and lauric acids, polymerized polyvinyl alcohol, self-emulsifying wax and non-denatured collagen.

4. Compositions as claimed in claim 1, characterised by being prepared in the form of oil-in-water emulsions, water-in-oil emulsions, hydrogels, pastes, ointments, lotions and powders for sprinkling.

5. Compositions as claimed in claim 1, characterised by a hyaluronic acid sodium salt content of between 0.1 and 0.5% by weight and a disinfectant substance content of between 0.1 and 2.0% by weight.

6. Compositions as claimed in claim 1, characterised by a pH of between 4 and 7.

7. Compositions as claimed in claim 1, characterised in that when prepared in the form of a water-in-oil emulsion they have an HLB of between 1 and 5.

8. Compositions as claimed in claim 1, characterised in that when prepared in the form of an oil-in-water emulsion they have an HLB of between 6 and 21.

## Patentansprüche

1. Pharmazeutische Zusammensetzungen zur äußerlichen Anwendung, umfassend Hyaluronsäure-Natriumsalz und desinfizierende Substanzen, ausgewählt aus der Gruppe bestehend aus Cresol-Derivaten, Hexetidin und Sulfadiazin-Silbersalz.

2. Zusammensetzungen gemäß Anspruch 1,
dadurch **gekennzeichnet,**
daß die Cresol-Derivate Chlorxylenol und Dichlorxylenol sind.

3. Zusammensetzungen gemäß Anspruch 1,
dadurch **gekennzeichnet,**
daß sie als Trägerstoffe und Diluentien Hydroxypropylmethylcellulose, Sorbitol, Glycerin, polyoxyethylenierte glykolysierte Glyceride, Polyethylenglykolstearat, Stearinsäure, Ölsäuredecylester, Capryl- und Capronsäureester, ethoxylierte Glyceride der Palmitin- und Laurinsäure, polymerisierter Polyvinylalkohol, selbstemulgierendes Wachs und nichtdenaturiertes Collagen umfassen.

4. Zusammensetzungen gemäß Anspruch 1,
dadurch **gekennzeichnet,**
daß sie in der Form von Öl-in-Wasser-Emulsionen, Wasser-in-Öl-Emulsionen, Hydrogelen, Pasten, Salben, Lotionen und Sprühpulvern zubereitet werden.

5. Zusammensetzungen gemäß Anspruch 1,
dadurch **gekennzeichnet,**
daß der Gehalt an Hyaluronsäure-Natriumsalz zwischen 0,1 und 0,5 Gew.-% und der Gehalt an desinfizierender Substanz zwischen 0,1 und 2,0 Gew.-% ist.

6. Zusammensetzungen gemäß Anspruch 1,
dadurch **gekennzeichnet,**
daß der pH-Wert zwischen 4 und 7 beträgt.

7. Zusammensetzungen gemäß Anspruch 1,
dadurch **gekennzeichnet,**
daß sie, wenn man sie in der Form einer Wasser-in-Öl-Emulsion zubereitet, einen HLB-Wert von zwischen 1 und 5 haben.

8. Zusammensetzungen gemäß Anspruch 1,
dadurch **gekennzeichnet,**
daß sie, wenn man sie in der Form einer Öl-in-Wasser-Emulsion zubereitet, einen HLB-Wert von zwischen 6 und 21 haben.

## Revendications

1. Compositions pharmaceutiques destinées à une utilisation locale et comprenant le sel de sodium d'acide hyaluronique et des désinfectants choisis dans le groupe constitué par : dérivés du crésol, hexétidine et sel d'argent de sulfadiazine.

2. Compositions selon la revendication 1, caractérisées en ce que lesdits dérivés du crésol sont le chloroxylénol et le dichloroxylénol.

3. Compositions selon la revendication 1, caractérisées en ce qu'elles comprennent, comme excipient et diluant : hydroxypropylméthylcellulose, sorbitol, glycérine, glycérides glycolysés polyoxyéthylés, stéarate de polyéthylèneglycol, acide stéarique, ester décylique d'acide oléique, esters d'acide caprylique et d'acide caproïque, glycérides éthoxylés d'acide palmitique et d'acide laurique, alcool polyvinylique polymérisé, cire auto-émulsionnante et collagène non dénaturé.

4. Compositions selon la revendication 1, caractérisées en ce qu'elles sont préparées sous la forme d'émulsion huile-dans-eau, d'émulsion eau-dans-huile, d'hydrogel, de pâtes, d'onguents, de lotions et de poudres à saupoudrer.

5. Compositions selon la revendication 1, caractérisées par une teneur en sel de sodium d'acide hyaluronique comprise entre 0,1 et 5 % en poids et une teneur en désinfectant comprise entre 0,1 et 2,0 % en poids.

6. Compositions selon la revendication 1, caractérisées par un pH entre 4 et 7.

7. Compositions selon la revendication 1, caractérisées en ce lorsqu'on les prépare sous la forme d'une émulsion eau-dans-huile, leur indice HLB est compris entre 1 et 5.

8. Compositions selon la revendication 1, caractérisées en ce que, lorsqu'on les prépare sous la forme d'une émulsion huile-dans-eau, leur indice HLB est compris entre 6 et 21.
